# EUROPEAN PATENT APPLICATION

(11) **EP 0 900 784 A1**
(43) Date of publication of application: **10.03.1999**
(21) Application number: 98810818.9
(22) Date of filing: 21.08.1998
(51) Int. Cl.: C07C 309/32, C11D 3/42, C11D 9/44, D06L 3/12, D21H 21/30

(54) **Sulphonated distyryl-biphenyl compounds**

(30) Priority: 30.08.1997 GB 9718353
(71) Applicant: Ciba Specialty Chemicals Holding Inc., 4057 Basel (CH)
(72) Inventor: Reinehr, Dieter, 79400 Kandern (DE); Sauter, Hanspeter, 79650 Schopfheim (DE); Kramer, Johannes, 5073 Gipf-Oberfrick (CH); Weigmann, Reinhard, 79400 Kandern (DE); Eckhardt, Claude 16, 68400 Riedisheim (FR); Meyer, Hans Rudolf, 4102 Binningen (CH)

(57) **Abstract**

Sulphonated distyryl-biphenyl compounds of the formula: 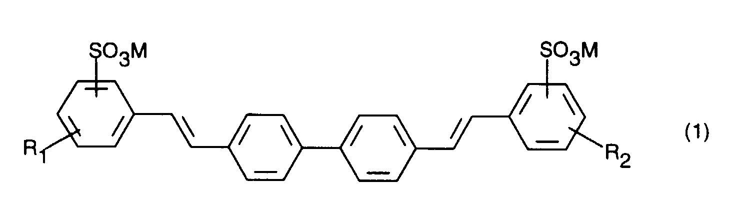 in which R₁ and R₂, independently, are hydrogen, 1-5 C- alkyl, 1-5 C- alkoxy or halogen, M represents hydrogen, an alkaline- or alkaline earth-metal, or ammonium, and the two sulphonic acid groups are in the 2,3'-, 2,4'- or 3,4'-positions of the respective benzene rings, with the proviso that when the sulphonic acid groups are in the 2- and 3'-positions, R₁ is not hydrogen and R₂ not a 4'- chloro- substituent and mixtures of these compounds together with symmetrically sulphonated distyryl-biphenyls as optical brightening agents for textiles, detergents and paper.

## Description

The present invention relates to new asymmetrically sulphonated distyryl-biphenyl compounds and to mixtures of these compounds together with known symmetrically sulphonated distyryl-biphenyls.

Known symmetrically sulphonated distyryl-biphenyls are, for example, 4,4'-bis-(2-sulphostyryl)-biphenyl from British Patent Specification 1,247,934 and 4,4'-bis-(4-chloro-3-sulphostyryl)-biphenyl from French Patent Specification 2,168,210. Combinations of these 2 compounds and their use as fluorescent whitening agents in detergent compositions have also been disclosed in US Patent Specification 4,147,648. Despite the excellent whitening effects attainable with such compounds and mixtures, it has now been found that the compounds of the present invention and their mixtures exhibit outstanding effects, particularly with respect to their efficiencies of whitening.

Sulphonated distyryl-biphenyl compounds of the present invention are represented by the formula: 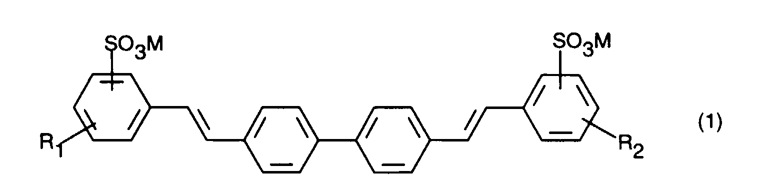 in which R₁ and R₂, independently, are hydrogen, 1-5 C- alkyl, 1-5 C- alkoxy or halogen, M represents hydrogen, an alkaline- or alkaline earth-metal, or ammonium, and the two sulphonic acid groups are in the 2,3'-, 2,4'- or 3,4'-positions of the respective benzene rings, with the proviso that when the sulphonic acid groups are in the 2- and 3'-positions, R₁ is not hydrogen and R₂ not a 4'- chloro- substituent.

Preferably, in the compounds of formula (1) or mixtures thereof, R₁ and R₂ are identical.

Still further preferred compounds of the present invention are those in which, in formula (1), R₁ and R₂ are hydrogen, or chlorine, and M represents hydrogen, potassium or sodium.

Especially preferable compounds of the present invention are of the formulae: 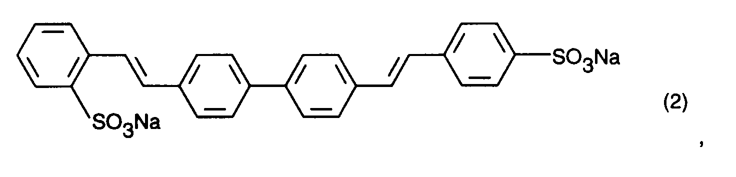 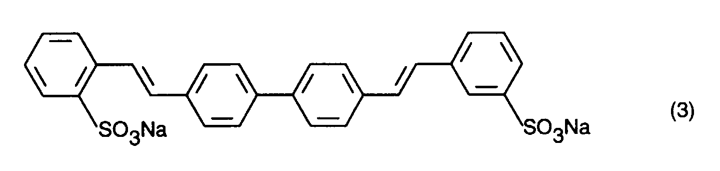 and

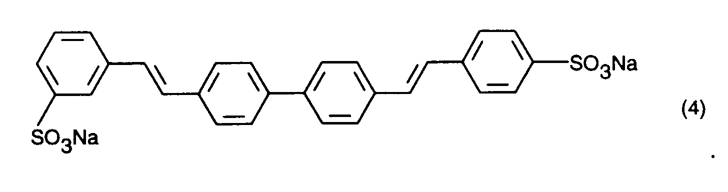

A further subject of the present invention are mixtures of compounds of formula (1), optionally together with 1 or more symmetrically substituted sulphonated distyryl-biphenyl compounds, in which the sulphonic acid groups are at the 2,2'-, 3,3'- or 4,4'-positions of the respective benzene rings of formula (1). Preferably, such mixtures contain 1 to 3 symmetrically substituted sulphonated distyryl-biphenyl compounds.

Especially preferred mixtures are those comprising at least one compound of the following formulae (2) - (4) combined either with each other and/or with one or more of the compounds of the following formulae (5) - (7): 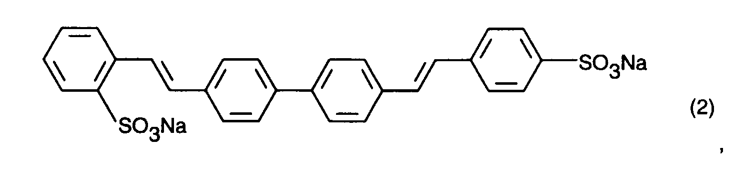 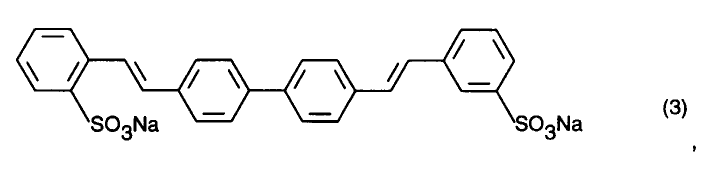 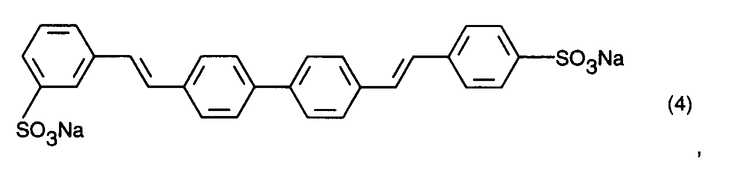 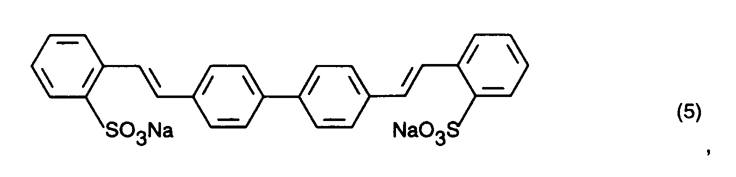 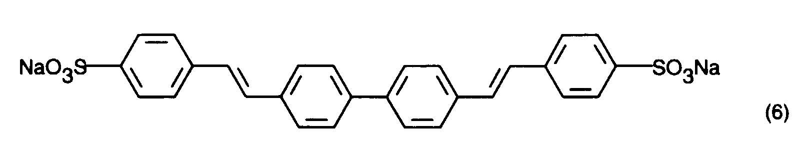 and

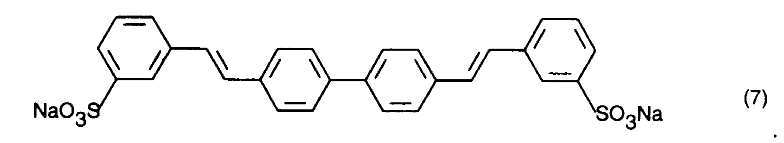

The composition of these mixtures is arbitrary. The ratio of their components may vary within broad limits. The ratios of mixtures containing the compound of formulae (3), (5) and (7) are e.g. (25-50) : (5-40) : (15-60).
Representative mixtures contain compound of the formulae (2), (5) and (6) with a ratio of e.g. 30:65:5, and of the formulae (3), (5) and (7) with ratios of e.g. 36:7:57 and 45:34:21.

When, in formula (1), R₁ and R₂ represent 1-5 C-alkyl, these may be methyl, ethyl, n- or isopropyl, n-, sec-,or t-butyl, n-pentyl, iso-amyl or sec-amyl groups. When, in formula (1), R₁ and R₂ represent 1-5 C-alkoxy, these may be methoxy, ethoxy, n- or isopropoxy, n-, sec-,or t-butoxy, n-pentyloxy, iso-amyloxy or sec-amyloxy groups. When, in formula (1), R₁ and R₂ represent halogen, these may be fluorine, chlorine, bromine, or iodine, preferably chlorine.

The compounds and/or mixtures of the present invention can be manufactured by methods which are themselves known, thus, for example, by reacting a compound of the formula 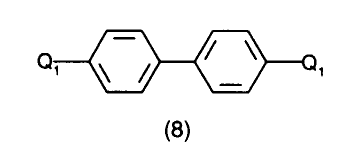 with each of the compounds of formulae 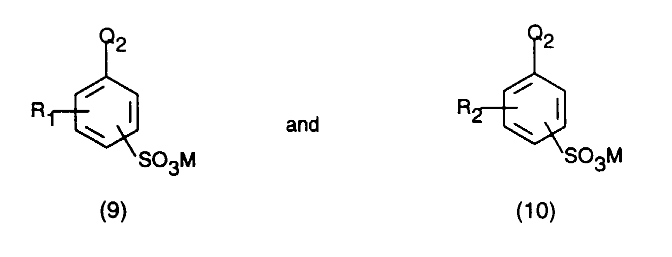 in which R_{1,} R₂ and M are as defined in claim 1, and the symbols Q₁ and Q₂ denote either a -CHO group or one of the groupings of the formulae 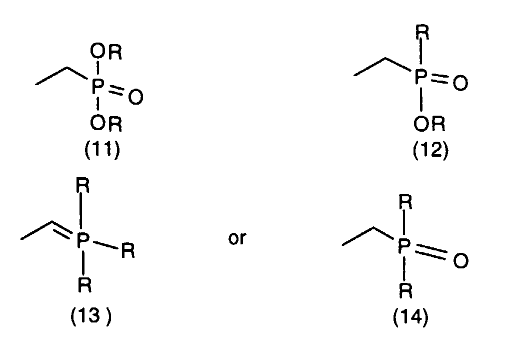 in which R represents an unsubstituted or substituted alkyl, aryl, cycloalkyl or aralkyl radical, in an aprotic solvent, in the presence of a strong base.

Preferably, Q₁ in formula (8) is represented by a grouping of one of the formulae (11), (12), (13) or (14), and Q₂ in formulae (9) and (10) is a -CHO group, and the group R in formulae (11), (12), (13) or (14) is a 1-5 C-alkyl, as defined above, phenyl, cyclohexyl or benzyl radical.

The aprotic solvent used in the above-described process may be an aromatic solvent, for example, benzene, toluene, xylene, a chlorobenzene or preferably a cyclic or linear amide such as N-methylpyrrolidone, dimethylformamide, diethylformamide or dimethylacetamide, or a sulphoxide such as dimethylsulphoxide.

Suitable strong bases are alkali metal hdroxides, amides and alcoholates. Particularly suitable are the lithium, sodium or potassium alcoholates containing 1-5 carbon atoms. In principle the reaction temperature can vary from 10°C to the boiling point of the solvent utilised, although it is generally advantageous to carry out the reaction within a temperature range of 30° to 60°C.

In dissolved or finely divided states, the brighteners defined above display a more or less pronounced fluorescence. They are therefore used, according to the invention, for optically brightening synthetic or natural organic materials.

Examples of such materials which may be mentioned, without the review given below being intended to express any limitation thereto, are textile fibres from the following groups of organic materials, insofar as optical brightening thereof enters into consideration:
(a) Polyamides which are obtainable as polymerisation products by ring opening, for example those of the polycaprolactam type,
(b) polyamides which are obtainable as polycondensation products based on bifunctional or polyfunctional compounds capable of undergoing a condensation reaction, such as hexamethylenediamine adipate and
(c) natural textile organic materials of animal or vegetable origin, for example based on cellulose or proteins, such as cotton or wool, linen or silk.

The organic materials to be optically brightened can be in diverse stages of processing and are preferably finished textile products. They can, for example be in the form of hank goods, textile filaments, yarns, twisted yarns, nonwovens, felts, textile fabrics, textile composites or knitted fabrics.

The brighteners defined above are of particular importance for the treatment of textile fabrics. The treatment of textile substrates is advantageously carried out in an aqueous medium in which the particular optical brighteners are present in a finely divided form (suspensions, so-called microdispersions and in some cases solutions). Dispersing agents, stabilisers, wetting agents and further auxiliaries can optionally be added during the treatment.

The treatment is usually carried out at temperatures of from about 20° to 140°C, for example at the boiling point of the bath, or in the region thereof (about 90°C). For the finishing, according to the invention, of textile substrates it is also possible to use solutions or emulsions in organic solvents, as are used in dyeing practice in so-called solvent dyeing (pad-thermofix method and the exhaustion dyeing process in dyeing machines).

The new optical brighteners which can be used according to the present invention can also be employed, for example, in the following use forms:
(a) In mixtures with so-called "carriers", wetting agents, softeners, swelling agents, antioxidants, light stabilisers, heat stabilisers and chemical bleaching agents (chlorite bleach and bleaching bath additives).
(b) In mixtures with crosslinking agents and finishing agents (for example starch or synthetic finishing agents) and also in combination with very diverse textile finishing processes, especially synthetic resin finishes (for example crease resistant finishes such as "wash-and-wear", "permanent press" and "no-iron"), and also flame resistant finishes, soft handle finishes, anti-soiling finshes or anti-static finishes or antimicrobial finishes.
(c) As additives to various soaps and washing agents.
(d) In combination with other substances having an optical brightening action.

If the brightening process is combined with textile treatment or finishing methods, the combined treatment can in many cases advantageously be effected with the aid of corresponding stable formulations which contain the compounds having an optical brightening action in a concentration such that the desired brightening effect is obtained.

In certain cases, the full effect of the brightener is achieved by an after-treatment. This can be, for example, a chemical treatment (for example acid treatment), a thermal treatment (for example heat) or a combined chemical/heat treatment.

The amount of the new optical brighteners to be used according to the invention, relative to the material to be optically brightened, can vary within wide limits. A distinct and durable effect can already be achieved with vary small amounts and in certain cases, for example, with amounts of 0.03% by weight. However amounts of up to about 0.5% by weight can also be used. For most cases of interest in practice, amounts of between 0.05 and 0.5% by weight relative to the material to be brightened, are preferably of interest.

The new optical brighteners are also especially suitable as additives for washing baths or to industrial and household washing agents and they can be added in various ways. They are appropriately added to washing baths in the form of their solutions in water or organic solvents or also in a state of fine division as aqueous dispersions or slurries. They, or their components, are advantageously added to household or industrial washing agents at any phase of the manufacturing process of the washing agent, for example to the so-called "slurry" prior to spray-drying of the washing powder or during the preparation of liquid washing agent combinations. The compounds can be added both in the form of a solution or dispersion in water or other solvents and also without auxiliaries in the form of a dry brightener powder. However, they can also be sprayed, in the dissolved or pre-dispersed form, onto the finished washing agent.

Washing agents which can be used are the known mixtures of detergent substances, such as, for example, soap in the form of chips and powders, synthetic products, soluble salts of sulphonic acid half-esters of higher fatty alcohols, arylsulphonic acids, which are substituted by higher alkyl and /or polysubstituted by alkyl, carboxylic acid esters with alcohols of medium to higher molecular weight, fatty acid acylaminoalkyl- or aminoaryl-glycerol-sulphonates, phosphoric acid esters of fatty alcohols and the like. So-called "builders" which can be used are, for example, alkali metal polyphosphates and alkali metal polymetaphosphates, alkali metal pyrophosphates, alkali metal salts of carboxymethylcellulose and other "soil redeposition inhibitors", and also alkali metal silicates, alkali metal carbonates, alkali metal borates, alkali metal perborates, nitrilotriacetic acid, ethylenediamine-tetraacetic acid and foam stabilisers, such as alkanolamides of higher fatty acids. Furthermore, the washing agents can contain, for example: antistatic agents, superfatting skin protection agents, such as lanolin, enzymes, antimicrobial agents, perfumes and dyestuffs.

The new brighteners have the particular advantage that they are also effective in the presence of active chlorine donors, such as, for example, hypochlorite and can be used without substantial loss of the effects in washing baths with non-ionic washing agents, for example alkylphenol polyglycol ethers. Also in the presence of perborate or peracids and activators, for example tetraacetylglycoluril or ethylenediamine-tetraacetic acid are the new brighteners very stable both in pulverulent washing agent and in washing baths.

The brighteners according to the invention are added in amounts of 0.005 to 2% or more and preferably of 0.03 to 0.5%, relative to the weight of the liquid or pulverulent ready-to-use washing agent. When they are used to wash textiles made of cellulose fibres, polyamide fibres, cellulose fibres with a high grade finish, wool and the like, wash liquors which contain the indicated amounts of the optical brighteners according to the invention impart a brilliant appearance in daylight.

The washing treatment is carried out, for example, as follows:
The indicated textiles are treated for 1 to 30 minutes at 5° to 100°C and preferably at 25° to 100°C in a wash bath which contains 1 to 10 g/kg of a composite washing agent containing builders and 0.05 to 1% relative to the weight of the washing agent, of the brighteners claimed. The liquor ratio can be 1:3 to 1:50. After washing, the textiles are rinsed and dried in the customary manner. The wash bath can contain, as a bleach additive, 0.2 g/l of active chlorine (for example in the form of hypochlorite) or 0.1 to 2 g/l of sodium perborate.

The brighteners according to the invention can also be applied from a rinsing bath with a "carrier". For this purpose the brightener is incorporated in a soft rinsing agent or in another rinsing agent, which contains, as the "carrier", for example, polyvinyl alcohol, starch, copolymers on an acrylic basis or formaldehyde/urea or ethylene-urea or propylene-urea derivatives, in amounts of 0.005 to 5% or more and preferably of 0.2 to 2%, relative to the rinsing agent. When used in amounts of 1 to 100 ml, and preferably of 2 to 25 ml, per litre of rinsing bath, rinsing agents of this type, which contain the brighteners according to the invention, impart brilliant brightening effects to very diverse types of treated textiles.

A further application of the compounds of the invention is for the brightening of paper, either in the pulp mass during paper manufacture or in the size-press, which has been described in British Patent Specification 1,247,934, or preferably in coating compositions. Such coating compositions utilising certain distyryl-biphenyl optical brighteners have been described in British Patent Specification .2,277,749 in considerable detail. When brighteners of the present invention are employed in such formulations papers brightened with them exhibit a very high degree of whiteness.

The following Examples serve to illustrate the invention; parts and percentages are by weight, unless otherwise stated.

### Example 1

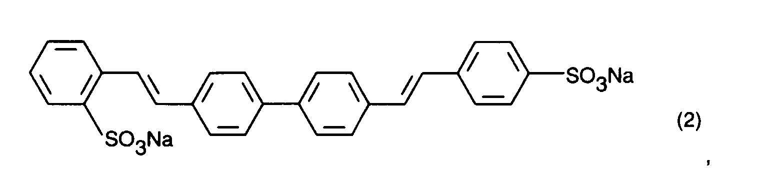

23.0 g. of the sodium salt of benzaldehyde-4-sulphonic acid , 31.13 g of 4,4'-bis-(dimethoxyphophonomethyl)-diphenyl and 20.4 g of the sodium salt of benzaldehyde-2-sulphonic acid are dispersed in 300 ml. of anhydrous dimethylformamide under an atmosphere of nitrogen. After warming to 40°C with stirring, 32 g. of a 30% methanolic solution of sodium methylate are added over a period of 10 minutes. After stirring for a further 3 hours at 40-45°C, the mixture is cooled to room temperature, filtered and the filtrate washed with dimethylformamide. The filtrate is evaporated to dryness under vacuum and the residue (31.4 g) boiled in 120 ml. of methanol. After cooling to room temperature the solids are filtered off, washed with methanol and dried. There are obtained 11.3 g. of the compound of formula (2) as a beige powder, corresponding to a yield of 51.5%. This product is then boiled in 140 ml. of n-propanol and 60 ml. of water in the presence of 0.5 g. of activated charcoal, clarified hot and evaporated to yield 5.3 g. of a pale yellow product which, according to TLC and HPLC is pure.

| Analysis for C₂₈H₂₀Na₂O₆S₂.1.84 H₂O: | | | | | | |
|---|---|---|---|---|---|---|
| calculated | C 56.45%, | H 4.01%, | S 10.77%, | O 21.06%, | H₂O 5.56%, | Na 7.72% |
| found | C 56.25%, | H 4.07%, | S 10.78%, | O 21.37%, | H₂O 5.56%, | Na 7.75% |

### Example 2

7.4 g. of the sodium salt of benzaldehyde-2-sulphonic acid, 3.6 g. of the sodium salt of benzaldehyde-4-sulphonic acid and 8.4 g. of 4,4'-bis-(dimethoxyphophonomethyl)-diphenyl are added to 40 ml. of dimethylformamide, warmed to 40°C and treated with 8.7 g. of a 30% methanolic solution of sodium methylate over a period of 10 minutes. After stirring for a further 2 hours at 40-45°C the solution is evaporated to dryness, the residue (22 g.) dissolved in 80 ml. of hot water, treated with 8.9 g of salt, cooled and filtered. There are obtained 11.8 g. of a pale greenish yellow product containing 75% of a mixture of isomers (2), (5) and (6), corresponding to a total yield of 78.7% of theory.
The ratio of the isomers (2) : (5) : (6) is 30 : 65 :5 according to HPLC.

### Example 3

The procedure described in Example 1 is followed using the sodium salt of benzaldehyde-3-sulfonic acid instead of the sodium salt of benzaldehyde-4-sulfonic acid. The crude reaction product is worked up as indicated in Example 1. There is obtained a pale yellow product containing a mixture of the compounds of formulae (3), (5) and (7).
The ratio of the isomers of the formulae (3), (5) and (7) is 36:7:57 according to HPLC.

### Example 4

The procedure described in Example 3 is followed by reacting 4,4'-bis-(dimethoxyphosphonomethyl)-diphenyl and the sodium salt of benzaldehyde-2-sulfonic acid. After a reaction time of 3 hours at 40 to 45°C the sodium salt of benzaldehyde-3-sulfonic acid is added to the reaction mixture which is then stirred for a further 3 hours at 40 to 45°C. The crude reaction product is worked up as indicated. There is obtained a pale yellow product containing a mixture of the compounds of formulae (3), (5) and (7). The ratio of the isomers of formulae (3), (5) and (7) is 45:34:21 according to HPLC.

### Example 5

A standard (ECE) washing powder is made up from the following components in the indicated proportions (weight %):

| | |
|---|---|
| 8.0% | Sodium (C_{11.5})alkylbenzene sulfonate |
| 2.9% | Tallow alcohol-tetradecane-ethylene glycol ether (14 mols EO) |
| 3.5% | Sodium soap |
| 43.8% | Sodium tripolyphosphate |
| 7.5% | Sodium silicate |
| 1.9% | Magnesium silicate |
| 1.2% | Carboxymethyl cellulose |
| 0.2% | EDTA |
| 21.2% | Sodium sulfate |
| 0 or 0.1% | compound/compund mixture according to Examples 1 to 4 and Water to 100%. |

A wash liquor is prepared by dissolving 0.8 g. of the above washing powder in 200 mls. of tap water. 10 g. of bleached cotton fabric is added to the bath and washed at 25°C. over 15 minutes and then rinsed, spin-dried and ironed at 160°C. This washing procedure is repeated three and ten times.

After the third and tenth washes, the whiteness of the washed samples is measured with a DCI/SF 500 spectrophotometer according to the Ganz method.

The results obtained are set out in the following Table 1:

**Table 1**

| Example | Ganz Whiteness | |
|---|---|---|
| | 3 washes | 10 washes |
| control | 61 | 61 |
| 1 | 176 | 203 |
| 2 | 164 | 198 |
| 3 | 172 | 205 |
| 4 | 176 | 206 |

(control = 0% fluorescent whitening agent (fwa:); Examples 1 to 4 = 0.1% fwa.
The results in Table 1 demonstrate that washing with a detergent containing an inventive compound/compound mixture improves the Ganz Whiteness (GW) with successive washings.

## Claims

1. Sulphonated distyryl-biphenyl compounds of formula: 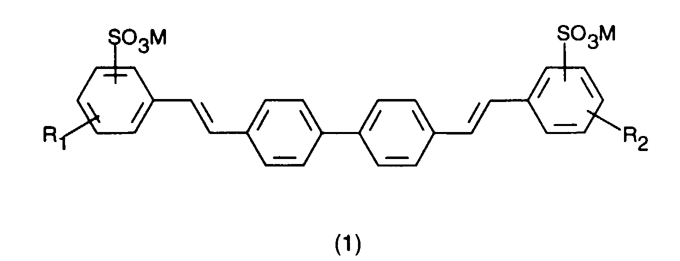 in which R₁ and R₂, independently, are hydrogen, 1-5 C- alkyl, 1-5 C- alkoxy or halogen, M represents hydrogen, an alkaline- or alkaline earth-metal, or ammonium, and the two sulphonic acid groups are in the 2,3'-, 2,4'- or 3,4'-positions of the respective benzene rings, with the proviso that when the sulphonic acid groups are in the 2- and 3'-positions, R₁ is not hydrogen and R₂ not a 4'- chloro- substituent

2. Compounds according to claim 1, or mixtures thereof, in which R₁ and R₂ are identical.

3. Compounds according to claim 2, in which R₁ and R₂ are hydrogen, or chlorine, and M represents hydrogen, potassium or sodium.

4. Compounds of the formulae: 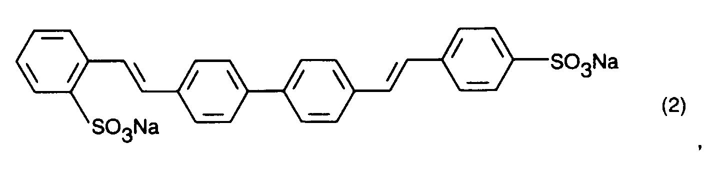 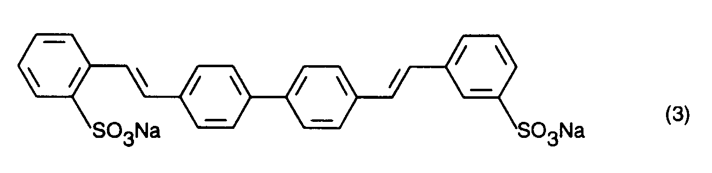 and

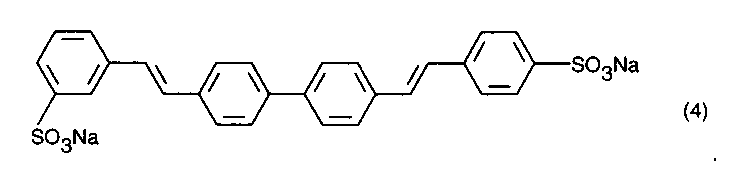

5. Mixtures of compounds according to claim 1, optionally together with 1 or more symmetrically substituted sulphonated distyryl-biphenyl compounds, in which the sulphonic acid groups are at the 2,2'-, 3,3'- or 4,4'-positions of the respective benzene rings of formula (1).

6. Mixtures of compounds according to claim 5, together with 1 to 3 symmetrically substituted sulphonated distyryl-biphenyl compounds,

7. A mixture according to claim 5, comprising at least 2 of the compounds (2) - (7) of the following formulae: 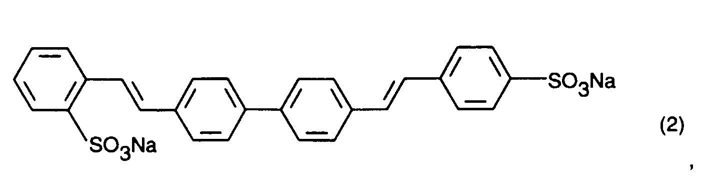 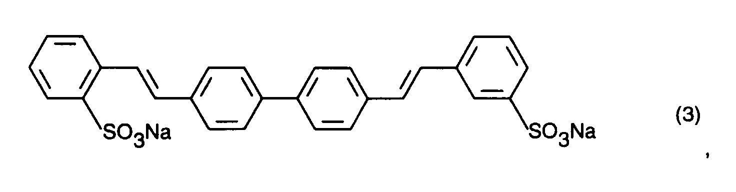 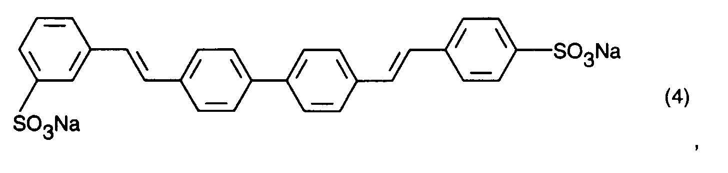 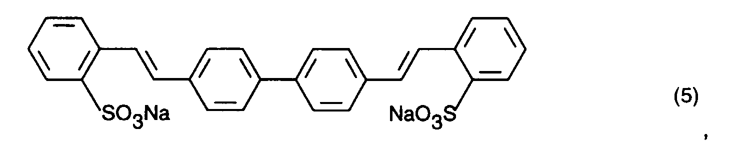 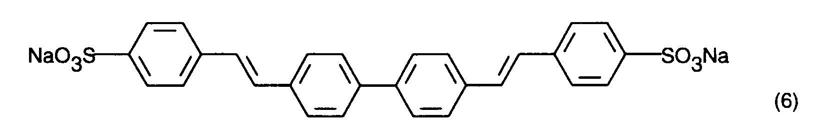 and

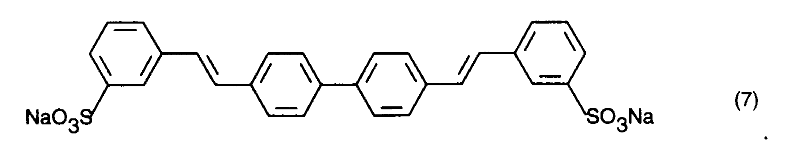

8. A process for the preparation of compounds according to claim 1 in which a compound of the formula 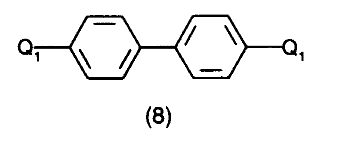 is reacted with each of the compounds of formulae 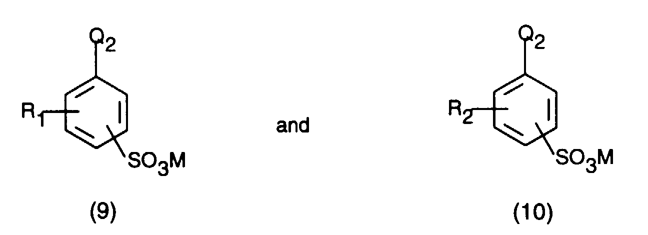 in which R_{1,} R₂ and M are as defined in claim 1, and the symbols Q₁ and Q₂ denote either a -CHO group or one of the groupings of the formulae 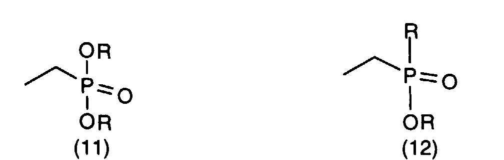 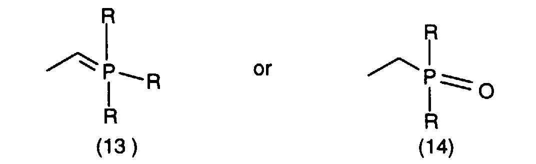 in which R represents an unsubstituted or substituted alkyl, aryl, cycloalkyl or aralkyl radical, in an aprotic solvent, in the presence of a strong base.

9. A process according to claim 8 in which Q₁ formula (8) is represented by a grouping of one of the formulae (11), (12), (13) or (14), and Q2 in formulae (9) and (10) is a -CHO group.

10. A process according to claim 9 in which the group R in formulae (11), (12), (13) or (14) is a 1-5 C-alkyl, phenyl. cyclohexyl or benzyl radical.

11. An optical brightening composition which comprises at least one distyryl-biphenyl compound as claimed in any one of claims 1 to 4 or a mixture as claimed in any one of claims 5 to 7.

12. A soap or detergent composition according to claim 11.

13. A composition according to claim 11 for the optical brightening of textile fibres.

14. A composition according to claim 11 for the optical brightening of paper.

15. A method for whitening a textile or paper substrate comprising applying to the substrate any of the compounds according to any one of claims 1 to 4 or any of the compound mixtures according to any one of claims 5 to 7.

16. Use of any of the compounds according to any one of claims 1 to 4 or any of the compound mixtures according to any one of claims 5 to 7 for optically whitening a textile or paper substrate.
